# EUROPÄISCHE PATENTSCHRIFT

(11) **EP 1 859 733 B1**
(45) Veröffentlichungstag und Bekanntmachung des Hinweises auf die Patenterteilung: **29.05.2019**
(21) Anmeldenummer: 07010126.6
(22) Anmeldetag: 22.05.2007
(51) Int. Cl.: A61B 5/0205, A61B 5/0432

(54) **Verfahren und Vorrichtung zur Korrelation von Signalen der Atmung des Herz-Kreislauf-Systems**
Method and device for correlating breathing signals in the cardiovascular system
Procédé et dispositif destinés à mettre en corrélation les signaux de respiration du système cardiocirculatoire

(30) Priorität: 24.05.2006 DE 102006024908
(43) Veröffentlichungstag der Anmeldung: 28.11.2007
(73) Patentinhaber: Löwenstein Medical Technology S.A., 2557 Luxembourg (LU)
(72) Erfinder: Schwaibold, Matthias, D-76137 Karlsruhe (DE); Schöller, Bernd, D-76135 Karlsruhe (DE)
(74) Vertreter: Klickow & Wetzel PartGmbB

(56) Entgegenhaltungen:
- WO-A-01/91631
- DE-A1-102004 042 797
- US-A- 4 276 888
- US-A1- 2003 004 423
- US-A1- 2005 115 561
- US-B1- 6 306 088

## Beschreibung

Die Erfindung betrifft ein Verfahren zur Darstellung und Auswertung von EKG-Signalen und atmungsabhängigen Signalen in Form einer vergleichenden Darstellung der verschiedenen Kanäle miteinander, wobei die Daten zeitlich zueinander korreliert abgespeichert werden, sowie bei dem für die Erkennung eines Artefaktes eines Herzschrittmachers eine EKG-Ableitung durchgeführt wird, wobei eine kontinuierliche Aufzeichnung über zumindest sechs Stunden bei simultaner und mindestens zwei-kanaliger EKG-Ableitung durchgeführt wird, und bei dem zur Übertragung der Messdaten der verschiedenen Sensoren an die Auswertungseinrichtung (17) getrennte Kanäle verwendet werden, wobei zur Konfigurierung eines Zeitraumes der Erfassung und Aufzeichnung innerhalb der gesamten Messdauer für jeden Kanal einzeln oder für mehrere Kanäle gebündelt eine Konfigurationsmöglichkeit verwendet wird.

Die Erfindung betrifft darüber hinaus eine Datenerfassungsanordnung zur Überwachung zumindest zweier physiologischer Parameter eines Patienten, nämlich mindestens eines Herzaktionsparameters mittels eines Meßverfahrens ausgewählt aus der Gruppe
a) EKG (Elektrokardiogramm) mit mindestens 2 Kanälen und einer Schrittmachererkennung,
b) Fingerpulsamplitude (FPA),
   und mindestens eines Atmungsparameters mittels eines Meßverfahrens ausgewählt aus einer Gruppe
c) Pulsoxymetrie,
d) respiratorische Polygraphie,
e) Polysomnografie
wobei die Datenerfassungsanordnung jeden physiologischen Parameter in einem separaten Kanal aufzeichnet und aufeinanderfolgende Werte der physiologischen Variablen speichert zum Gewinnen einer graphischen Darstellung oder Auswertung der gespeicherten Werte, wobei eine Takteinrichtung ein Zeitsignal erzeugt und eine Steuereinrichtung der Datenerfassungsanordnung während des Zeitsignals alle Kanäle der Datenerfassungsanordnung aufzeichnet und jedem Datensignal ein Zeitsignal zuordnet.

Die Früherkennung kardiorespiratorischer Krankheiten und die Identifikation ihrer Ursache als obstruktive oder zentrale Störung sind bedeutend für die erfolgreiche Behandlung und therapeutische Begleitung. Beispiele hierfür sind die Früherkennung von Asthma, die Beobachtung chronischobstruktiver Lungenerkrankungen sowie die Darstellung der Auswirkungen obstruktiver oder zentraler Schlafapnoe auf das Herz-/Kreislaufsystem.

Patienten mit krankhafter Sauerstoffentsättigung weisen von Seiten der Sauerstoffversorgung kardiale Belastungszeichen auf. Aus Atmungsstörungen können sich kardiale Störungen entwickeln. Bei Verdacht auf schlafbezogene Atmungsstörungen werden die respiratorische Polygraphie und / oder die Polysomnographie durchgeführt. Entsprechende Vorrichtungen sind lange bekannt. Vorrichtungen zur Aufzeichnung und Analyse von Signalen der Herztätigkeit sind ebenfalls bekannt.

Die Überwachung der Vitalparameter der Atmung und des Herz-Kreislauf-Systems und eine damit verbundene Auswertung des Zustands sowie die Warnung vor spontan auftretenden oder zu erwartenden Gefahren für Leben und Gesundheit ist daher wünschenswert. Ziel ist, bei lebensbedrohenden Zuständen rechtzeitig zu warnen oder im schlimmsten Fall Herz- beziehungsweise Atemstillstände zu registrieren und sofort zu alarmieren, um Akut-Maßnahmen einzuleiten. Der apparative Aufwand für die kontinuierliche Überwachung ist beim heutigen Stand der Technik jedoch erheblich. Bisher ist es üblich, die Signale der einzelnen Sensoren getrennt voneinander zu betrachten und zu bewerten. Signale der Atmung und Signale des Herzens werden aufgenommen, aber nie korreliert.

In der WO 01/91631 wird bereits eine Datenerfassungsanordnung zur Überwachung von mindestens zwei physiologischen Parametern eines Patienten beschrieben. Die entsprechenden Parameter werden jeweils in einem separaten Kanal aufgezeichnet und zur Bereitstellung einer graphischen Darstellung abgespeichert. Von einer Takteinrichtung wird ein Zeitsignal derart erzeugt, daß jedem Datensignal ein Zeitsignal zuordbar ist.

Aus der US 2005/115561 ist ein Verfahren zur Darstellung und Auswertung von EKG-Signalen sowie atmungsabhängigen Signalen bekannt. Es wird eine Abspeicherung von Daten sowie eine Anzeige der unterschiedlichen Kanälen zugeordneten Daten durchgeführt.

Die US 4,276,888 beschreibt eine weitere Meß- und Anzeigeeinrichtung für vitale kardiologische Parameter und Atmungsparameter.

In der US 2003/004423 werden ein Verfahren und eine Vorrichtung zur nicht invasiven Ermittlung und Anzeige von Schlafparametern beschrieben.

Die DE 10 2004 042 797 beschreibt ein Gerät zur Erfassung und Signalisierung von Schlafstörungen. Es werden mehrere Meßparameter in getrennten Kanälen erfaßt und Übertragen.

Die US 6,306,088 beschreibt eine mobile Einrichtung zur Erfassung von Körperparametern eines Patienten, bei der eine Mehrzahl von Sensoren am Körper des Patienten angeordnet werden. Die Meßdaten werden zu einer getrennt vom Patienten angeordneten Verarbeitungs- und Anzeigeeinrichtung übertragen, die auch zur Abspeicherung der Meßwerte ausgebildet ist.

Aufgabe dieser Erfindung ist es, ein Verfahren und eine Vorrichtung bereitzustellen, anhand der eine einfache und schnelle Korrelation von Signalen der Atmung und des Herz-Kreislauf-Systems ermöglicht wird.

Diese erste Aufgabe wird erfindungsgemäß dadurch gelöst, daß für die Erkennung eines Artefaktes eines Herzschrittmachers eine EKG-Ableitung durchgeführt wird, wobei eine kontinuierliche Aufzeichnung über zumindest sechs Stunden bei simultaner und mindestens zwei-kanaliger EKG-Ableitung durchgeführt wird, und daß zur Übertragung der Meßdaten der verschiedenen Sensoren an die Auswertungseinrichtung getrennte Kanäle verwendet werden, wobei zur Konfigurierung eines Zeitraumes der Erfassung und Aufzeichnung innerhalb der gesamten Meßdauer für jeden Kanal einzeln oder für mehrere Kanäle gebündelt eine Konfigurationsmöglichkeit verwendet wird.

Nach dem Grundgedanken der Erfindung erfolgt eine kontinuierliche Erfassung von physiologischen Parametern, wie beispielsweise Atmung, Atemfluss, Atemanstrengung, EKG, Herzrate, Temperatur, Feuchtigkeit an der Hautoberfläche, Sauerstoffsättigung, Blutdruck, EOG, EEG, EMG, die gemeinsam betrachtet eine Einschätzung des Gesundheitszustandes beziehungsweise eine kontinuierliche Überwachung und eine Warnung vor pathophysiologischen Zusammenhängen der Systeme Atmung - Schlaf - Herz/Kreislauf sowie eventuell kurz- oder langfristig lebensbedrohenden Zuständen ermöglichen können oder aber auch die Entstehung und Entwicklung von Krankheiten frühzeitig zu erkennen erlauben können.

Der Erfindung liegt weiterhin die Aufgabe zugrunde, eine Vorrichtung zu schaffen, die eine zeitgetaktete Aufzeichnung und Analyse von Signalen der Herztätigkeit und der Atmung ermöglicht, zur Ermittlung von ursächlichen Zusammenhängen die beiden großen vitalen Systeme betreffend.

Erfindungsgemäß wird zur Lösung der Aufgabe die Datenerfassungsanordnung der einleitend genannten Art derart weitergebildet, daß zur Übertragung der Meßdaten der verschiedenen Sensoren an die Auswertungseinrichtung getrennte Kanäle verwendet sind, wobei zur Konfigurierung eines Zeitraumes der Erfassung und Aufzeichnung innerhalb der gesamten Meßdauer für jeden Kanal einzeln oder für mehrere Kanäle gebündelt eine Konfigurationsmöglichkeit verwendet ist.Die Daten der einzelnen Kanäle werden in einer Speichereinrichtung ablegt. Eine Signalverarbeitungseinheit detektiert sowohl Parameter eines typischen Langzeit-EKG-Systems (Arrhythmieanalyse, morphologische Analyse, Frequenz- und Zeitvariationen) als auch Atmungs- und/oder Schlafstörungen und gibt diese über eine Visualisierungseinheit dem Benutzer aus. Durch eine - wiederaufladbare - Batterie wird eine mobile, netzungebundene Aufzeichnung der Signale für mindestens 2 Stunden möglich.

Nachfolgend werden Ausführungsbeispiele beschrieben.
- Fig. 1: zeigt eine schematische Darstellung der erfindungsgemäßen Vorrichtung,
- Fig. 2: zeigt eine beispielhafte Aufzeichnung von fünf physiologischen Kanälen,
- Fig. 3: zeigt eine schematische Darstellung der erfindungsgemäßen Vorrichtung mit optionalen Peripheriegeräten und
- Fig. 4: eine Darstellung einer Bedienoberfläche einer Software zur Auswertung und Verwaltung der registrierten Daten.

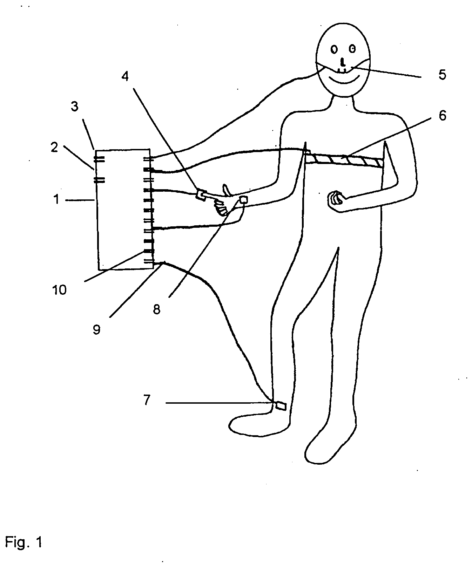

In Fig. 1 ist die Vorrichtung zur Erfassung der physiologischen Parameter (1) dargestellt. Über Schnittstellen (2) und (3) sind externe Geräte wie ein PC, ein Drucker oder ein Therapiegerät anschließbar. Verschiedene Sensoren werden über Steckkontakte (10) mittels Kabel oder Funk an die Vorrichtung zur Erfassung der physiologischen Parameter angeschlossen. Vorgesehen sind zumindest ein Pulsoximeter (4) zur Erfassung der Sauerstoffsättigung, ein Sensor zur Erfassung des Atemstroms (5), ein Gurt zur Erfassung von Köperbewegungen (6) sowie drei Elektroden ((7) und (8) zur Erfassung von elektrischen Signalen, hier zwei EKGs.

Die Messdaten der verschiedenen Sensoren werden über getrennte Kanäle einer gemeinsamen Auswertungseinrichtung (17) bereitgestellt. Dabei ist es nicht notwendig, sämtliche Kanäle über die gesamte Messzeit aufzuzeichnen. Vielmehr bietet die Vorrichtung eine Konfigurationsmöglichkeit, über die der Benutzer für jeden Kanal einzeln oder für mehrere Kanäle gebündelt den Zeitraum der Erfassung und Aufzeichnung innerhalb der gesamten Messdauer festlegen kann.

Die Sensoren sind kabelgebunden mit der Auswertungseinrichtung verbunden. Von dieser wird die Stromversorgung für die Sensoren bereitgestellt und dort werden die Daten aufbereitet und ggf. digital an den Computer (19) weitergegeben. Dieser visualisiert die Messkanäle und speichert die eingehenden Daten.

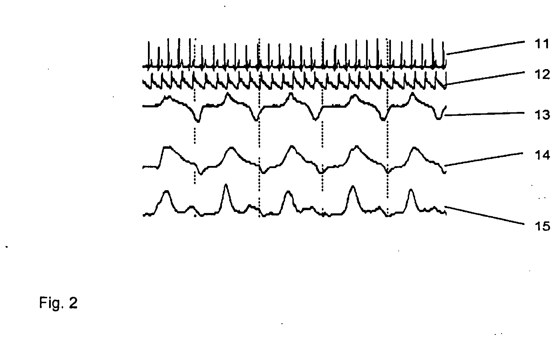

In Fig. 2 ist die Visualisierung der Messkanäle dargestellt. In Spur (11) ist das EKG-Signal, das über die zwei Elektroden (7) und (8) aufgezeichnet wurde, dargestellt. In Spur (12) ist das Plethysmogramm des Pulsoximeters (4) dargestellt. Spur (13) zeigt den Atemstrom, wie er von dem Sensor (5) aufgezeichnet wurde. Die Bewegungen von Thorax (14) und Abdomen (15) werden über Piezo-Effort-Sensoren aufgezeichnet (6).

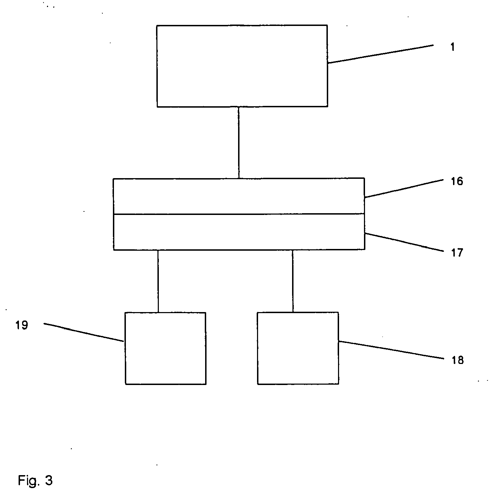

In Fig. 3 ist dargestellt, wie die Meßdaten von der Vorrichtung zur Erfassung der physiologischen Parameter (1) durch eine Auswertungseinrichtung (17) analysiert werden und im Bereich der Speichereinrichtung (16) gespeichert werden. Über Schnittstellen können die Daten an externe Geräte wie einen PC (18) oder ein Beatmungsgerät-Gerät (19) weitergegeben werden.

Erfindungsgemäß ist daran gedacht, die Funktion bestehender Langzeit-EKG Geräte mit der bestehender Polysomnographie-Geräte in EINEM Gerät zu verbinden. Dies dient der Erkennung von ursächlichen Zusammenhängen von beispielsweise Arrhythmie oder Ischämie des Herzens aufgrund einer Atmungs- und/oder Schlafstörung.

Die Kopplung mit einem Beatmungsgerät erlaubt es, bei Online-Erkennung von Ereignissen (Atmung, Sättigung, Schlaf, CO2, ...) eine Fernsteuerung des Beatmungsgerätes im Sinne einer automatischen Titration zu realisieren.

Erfindungsgemäß ist für den EKG-Kanal eine Schrittmachererkennung vorgesehen. Wenn der Patient einen Herzschrittmacher hat, muß das Artefakt des Schrittmachers durch das EKG erkannt werden. Dazu sind zwei Möglichkeiten vorgesehen:
a) Erhöhen der Abtastrate über 2 KHz bei einer digitalen Signalaufzeichnung,
b) Schaltung (analog), die am Ausgang einen zeitlich langen Spannungswert bereitstellt, der auch bei einer Abtastrate von 256 Hz oder niedriger als 2 KHz ermittelt werden kann.

In einem zusätzlichen Kanal wird die Schrittmachertätigkeit erfasst. Zumindest weist das erfindungsgemäße Gerät dazu 2 Kanäle auf.

Das erfindungsgemäße Gerät gewährleistet eine kontinuierliche Aufzeichnung über zumindest 6 Stunden, bevorzugt über 24h, bei simultaner, mindestens 2-kanaliger EKG-Ableitung.

Die kontinuierliche oder diskontinuierliche Auswertung stellt sicher, dass alle wichtigen Ereignisse erfaßt werden. Als wichtige Ereignisse gelten:
-- Asystolie über 2,0 sec. Dauer,
-- supraventrikuläre Tachykardie, Bradykardie,
-- Vorhofflimmern,
-- Vorhofflattern,
-- ventrikuläre Extrasystolen,
-- supraventrikuläre Extrasystolen
-- Biegeminus, Trigeminus, Pausen
-- höhergradige tachykarde ventrikuläre Rhythmusstörungen,
-- Kammertachykardie,
-- Kammerflattern,
-- Kammerflimmern,
-- sonstige Rhyhtmusstörungen
-- Herzfrequenzvariationen, Ereignisse mit Sympathikusaktivierung
-- Herzfrequenzvariabilität im Zeit- und Frequenzbereich
-- Poincaré-Diagramme
-- OSAS-Risikoindex aus dem EKG
-- Ischämie.

Der im Auswertesystem verfügbare Dokumentationsspeicher gewährleistet, daß auch bei gehäuft auftretenden Ereignissen eine in quantitativer Hinsicht korrekte Beurteilung möglich ist. Zudem verfügt das Gerät über eine Polygraphie und/oder Polysomnographie-Funktion.

Im Gerät werden zeitgetaktet alle Kanäle abgetastet. Dadurch werden die Informationen der Kanäle snychronisiert und zeit-assoziert auswertbar, ohne dass alle Kanäle dieselbe Abtastfrequenz besitzen müssen.

Die Auswertung erfolgt am PC und/oder im Gerät. Erfindungsgemäß ist somit eine Korrelation der Ereignisse möglich:
Beispielsweise wird ermittelt das Arrythmien oder Ischämien wegen oder zumindest zeitlich assoziiert mit Atmungsstörungen auftraten.

Beispielsweise kann ermittelt werden, dass von 300 Arrythmien oder Ischämien in 24 h, 200 atmungsbedingt auftraten.

Es ist eine unmittelbare Auswertung vorgesehen. Damit kann das Gerät unmittelbar auf Ereignisse reagieren und beispielsweise atmungsbedingt Arrythmien oder Ischämien durch Anpassung eines Therapiedrucks eines Beatmungsgerätes und/oder durch Sauerstoffgabe therapieren. Das Gerät gibt einen Alarm aus, wenn definierbar schwere Ereignisse auftreten.

Um störende Artefakte, wie beispielsweise ein mögliches 50 Hz Netzbrummen zu entfernen, wird ein 50 Hz Filter eingesetzt, um diese Störungen zu beseitigen. Des Weiteren wird nur ein Frequenzspektrum für die Analyse benötigt. Diese Abgrenzung erreicht man über einen Bandpass, durch den alle unrelevanten Frequenzen ausblendet werden. Nach diesen Basisfiltern kommt die eigentliche Analyse des Signals. Erfindungsgemäß ist die Vorrichtung geeignet, zeitgleich mindestens ein Herzaktions-, einen Atmungs- und einen Schlafparameter aufzuzeichnen und nächtliche Atmungsstörungen sowie definierbare EKG-Anomalien zu erkennen, sowie die erkannten EKG-Anomalien (Arrhythmien etc.) Wach- und/oder Schlaf-Zustand zuzuordnen sowie zeitliche Assoziationen zwischen EKG-Anomalien und Atmungsstörungen herzustellen, um einen ursächlichen Zusammenhang von beiden untersuchen zu können.

Die Vorrichtung besteht aus zumindest einer Sensoreinrichtung die zumindest einen Herzaktionsparameter, einen Atmungsparameter und einen Schlafparameter ermittelt, einer Zeitbestimmungseinrichtung, die eine Bestimmung von Zeitinformationen ermöglicht, einer Speichereinrichtung, die die ermittelten Biosignale und Zeitinformationen wieder abrufbar speichert und einer Vergleichseinrichtung, die auch nächtliche Atmungsstörungen sowie EKG-Anomalien erkennt, sowie die EKG-Anomalien (Arrhythmien etc.) Wach- und Schlaf-Zustand zuordnen kann sowie zeitliche Assoziationen zwischen EKG-Anomalien und Atmungsstörungen herstellen kann, um einen ursächlichen Zusammenhang von beiden untersuchen zu können.

Erfindungsgemäß ist vorgesehen, daß mindestens 2 der folgenden Messgrößen über einen Zeitraum von mindestens zwei Stunden simultan auf einem Datenträger registriert werden können:
1. Registrierung der Atmung
2. Oxymetrie (Sättigung des oxygenierbaren Hämoglobins)
3. Elektrokardiographie (EKG) mit mindestens 2 Kanälen
4. Aufzeichnung der Körperlage
5. Messung der abdominalen und thorakalen Atembewegungen
6. Atemfluss oder Maskendruckmessung (bei Überdrucktherapie mit CPAP- oder verwandten Geräten)
7. Elektrookulographie (EOG) mit mindestens 2 Ableitungen
8. Elektroenzephalographie (EEG) mit mindestens 2 Ableitungen
9. Elektromyographie (EMG) mit mindestens 3 Ableitungen
10. Optische und akustische Aufzeichnung des Schlafverhaltens

Erfindungsgemäß werden zumindest einige der folgenden Ereignisse oder Zustände erkannt
∘ Sinusrhythmus (P-Wellen vorhanden)
∘ Lagetyp (überdrehter Typ immer pathologisch)
∘ Extrasystolen, Arrhythmien allgemein
∘ Normalschlag, aktuelle und gemittelte Herzfrequenz
∘ AV-Block (PQ-Intervall normal < 0,2 s)
∘ Schenkelblock (QRS-Komplex normal < 0,1 s)
∘ Infarkt (ST-Hebung normal < 0,2 mV)
∘ T-Welle in I, II und V positiv (sonst nichttransmuraler Infarkt)
∘ QT-Intervall (länger als die halbe RR Zeit)
∘ Vorhofflimmern
∘ Vorhofflattern
∘ Lagetyp
∘ Vorhofhypertrophie
∘ Atrioventrikulärer Block
∘ WPW-Syndrom
∘ Schenkelblock
∘ QT-Syndrom
∘ Herzinfarkt
∘ Elektrolytstörungen
∘ Schnarchen
∘ nächtliche Atempausen
∘ schlafbezogene Atmungsstörungen
∘ obstruktive und zentralen Schlafapnoe- und Hypopnoe-Syndrome,
∘ obstruktive Rhonchopathien
∘ Apnoe- oder Hypopnoe-Phasen
∘ Sauerstoffentsättigungen des Blutes
∘ Schlafstadien
∘ kortikale oder subkortikale (autonome) Arousals

Erfindungsgemäß ist auch daran gedacht, im EKG Unterschiede zwischen Apnoikern und Gesunden zu finden, sowie auch Menschen mit Herzkrankheiten anhand ihres Herzrhythmus von Gesunden zu unterscheiden.

Ziel der Erfindung ist es auch, eine Vorrichtung anzugeben, welche nur anhand des EKGs REM- und Nicht-REM-Phasen voneinander unterscheidet. Bisher musste dazu in einem Schlaflabor ein erheblicher diagnostischer Aufwand getrieben werden. So könnte zum Beispiel ein Anästhesist mit Hilfe des EKGs die Ein- und Ausleitung einer Narkose überwachen. Umgekehrt kann man im EKG mit der neuen Methode auch deutlich erkennen, wann sich der Schläfer hin- und herwälzt. So ließe sich ein Maß dafür gewinnen, wie unruhig der Schlaf war.

Die computergestützte Signalanalyse von Meßdaten der Sensorsysteme zur Ableitung diagnostischer Informationen, beispielsweise bei kardiologischen Messungen wird, neben den klassischen Auswerteverfahren, basierend auf der Vermessung der Signale, zur Signalauswertung bei großen Datenmengen genutzt.

Erfindungsgemäß werden zur rechnergestützten Analyse und Interpretation der Atmungs- und EKG-Signalverläufen Einzelmerkmale bzw. Merkmalssätze identifiziert.

Die weitere Verarbeitung dieser Merkmalssätze zur Ausgabe von Befundhinweisen für den Arzt erfolgt mit Hilfe von Entscheidungsbäumen, neuronalen Netzen oder statistischen Verfahren. Die Richtigkeit und Vollständigkeit dieser Befunde hängen wesentlich von der Wahl der Merkmalssätze sowie der Genauigkeit der Parameterbestimmung ab. Durch den Einsatz neuer, rechenintensiver Algorithmen der Signalverarbeitung ist es möglich, eine Zerlegung der Signale in eine Vielzahl von Einzelparametern zu vermeiden und das ganzheitliche Signalmuster für die Analyse zu verwenden. Durch eine Verbindung dieser Algorithmen mit grossen Signalmusterbibliotheken kann die klassische Analyse, wie sie beispielsweise vom EKG bekannt ist, verbessert werden und es können darüber hinaus völlig neue Analysemethoden, die z.B. eine Trendaussage ermöglichen, geschaffen werden.

Die ausgewählten Signalmuster repräsentieren zumindest eine Periode des Zeittaktes. Der markierte Bereich wird signaltechnisch für die Korrelationsrechnung mit der Signaldatenbank aufbereitet.

Der Signalmustervergleich des markierten Bereiches mit der Signalmusterdatenbank erfolgt über die Berechnung der Korrelationsfunktion durch Verschiebung der Signalmuster der einzelnen Ableitungen von markiertem Bereich und Datenbank in der Zeitachse.

Im Ergebnis dieser Rechnungen erhält man für jeden Vergleich einen Vektor mit Korrelationsfunktionen, die entsprechend der besten Übereinstimmung zueinander bewertet werden. Da die Korrelationsfunktionen normiert berechnet werden, bedeutet der Korrelationswert 1 Übereinstimmung der Muster, während ein Wert 0 ausgeprägte Unähnlichkeit repräsentiert.

Da entsprechend der Ausgangshypothese nicht die ähnlichsten Meßwerte, sondern die ähnlichsten Signalmuster gesucht werden, müssen die vektoriellen Ergebnisse der Korrelationsrechnung zusammengefasst werden.

Durch die Zuordnung der bekannten Ereignisse der Datenbank zu den Korrelationsergebnissen der Signalmuster können Klassen mit unterschiedlichen Ereignisgruppen gebildet werden. Aus der Verteilung der Ereignisse der vom Signalmuster am besten übereinstimmenden Datenbank-Muster können Rückschlüsse auf das zu bewertende Signalmuster gezogen werden.

Auf diese Weise lässt sich mit Hilfe eines Signalmustervergleiches mit Hilfe einer Datenbank mit bekannten Ereignissen ein wahrscheinliches Ereignis für einen Messwert und/oder eine Vielzahl von Messwerten, die im gleichen Zeittakt aufgezeichnet wurden, angeben. Eine Zerlegung des Meßsignals in eine Anzahl von Signalparametern und Merkmalen ist nicht mehr erforderlich.

Erfindungsgemäß erfolgt die Realisierung des Verfahrens in vier aufeinanderfolgenden Verfahrensschritten:
1. Signal- aufzeichnung - aufbereitung
2. Mustervergleich
3. Klassifizierung
4. Auswertung und Aufbereitung der Klassifizierungsergebnisse für den Arzt sowie Bereitstellung diagnostischer und anderer Informationen mit Hilfe der Datenbank.

Dabei werden, vereinfacht gesagt, bekannte Muster mit den neu zu befundenden Mustern zum besseren Vergleich visuell "übereinander gelegt". Dies geschieht durch mathematische Mittelung, Ausrichtung in einem definierten Raster an der Null-Linie, präzise Justierung übereinander, Markierung der Wellengrenzen, farbliche Unterscheidung der Kurven und Zoomfunktion. Durch das Verfahren kann der Auswerter sofort, das heißt ohne Zeit- und Befundungsaufwand, relevante morphologische Veränderungen zwischen Bezugs- und aktuellem Meßwert erkennen und quantifizieren, insbesondere auch dann, wenn diese Veränderungen nur diskret sind oder die visuelle Auswertung durch Muskelartefakte, Netzbrummen und Basisliniendrift erschwert ist.

Die Integration der Funktionalität eines Langzeit-EKGs (Holter) in ein Poly(somno)graphiesystem ermöglicht eine umfassende somno-kardio-respiratorische Diagnose. Erfindungsgemäß werden ein Langzeit-EKG und ein Polygraphie- bzw. Polysomno-graphiesystem kombiniert, um den Zusammenhang von Schlafstörungen und kardiologischen Ereignissen aufdecken zu können. Mit diesem Gerät lassen sich sowohl Schlafuntersuchungen als auch Langzeit-EKG-Untersuchungen durchführen. Die Geräte-Software verfügt über alle notwendigen Funktionen und Analysen für eine 2-Kanal-Langzeit-EKG-Auswertung, d.h. Arrhythmieerkennung, Template-Verfahren zur Schlagklassifizierung, Ereignisklassifizierung und HRV-Analyse sowie Erstellung eines normgerechten Langzeit-EKG-Berichts. Aufgezeichnet werden die Ableitungen Einthoven I-II-III beispielsweise über 24 Stunden, die EKG-Analyse findet retrospektiv über zwei der drei Kanäle statt. Neben einer Auswertung des nächtlichen EKGs kann das System über Tag als reines Langzeit-EKG angewendet werden, sodass auch eine vollständige 24 h-Langzeit-EKG-Auswertung möglich ist.

Trotz des deutlich erhöhten Funktionsumfanges ist die PC-Software intuitiv und einfach bedienbar. Wichtig dabei ist, dass der typische Workflow bei der Auswertung eines Standard-Langzeit-EKGs genau so gut abgebildet wird wie der einer Poly(somno)grafie.

Nach Ablauf der beiden Einzel-Auswertungen bietet das System eine Aussage über die zeitliche (und ursächliche) Verknüpfung von EKG- sowie somno-respiratorischen Ereignissen. Dies geschieht
1) durch Übersichtsdarstellungen mit zeitlicher Markierung sämtlicher Ereignisse am Bildschirm und im Bericht,
2) durch statistische Aussagen über die Häufigkeit von Assoziationen zwischen EKG- und somno-respiratorischen Ereignissen im Bericht, zumindest beschränkt auf solche Verknüpfungen die plausibel erscheinen,
3) durch Differenzierung der Aussagen über EKG-Ereignisse und Herzfrequenzvariabilität nach Schlafstadien im Bericht.

Durch die Verknüpfung der Aussagen entsteht ein deutlicher Mehrwert gegenüber der Diagnose mit 2 Einzelgeräten an verschiedenen Tagen. Ziel ist, differentialdiagnostisch darstellen zu können, ob beim untersuchten Patienten rhythmologische EKG-Veränderungen durch Atmungs- bzw. Schlafstörungen ausgelöst werden, sowie die therapeutische Wirksamkeit einer PAP-Therapie im Einzelfall nachweisen zu können.

Alternative Ausbildungen der Erfindung umfassen:
- Eine vertiefte Auswertung des Zusammenhanges von EKG-Veränderungen und somno-respiratorischen Ereignissen
- Eine Erweiterung der EKG-Auswertung über den Basis-Umfang eines Holter-EKGs hinaus
- Ein 12-Kanal-Langzeit-EKG in einer Variante der Neuro-12-Box inklusive der dann um morphologische Aussagen erweiterten Auswertung
- Ein reines 2-Kanal-Langzeit-EKG ohne somnorespiratorische Kanäle mit Auswertung auf derselben PC-SW-Plattform mit zusätzlichem OSA/CSA-Screening-Parameter aus dem EKG.

Ausgangspunkt für die Analyse ist die Auswahl eines repräsentativen Signal-Bereiches durch Markierung. Entweder kann der Benutzer eine Markierung setzen oder es erfolgt eine automatische Markierung (Systemereignisse) von definierten Signalbereichen. Es wird daher zwischen Benutzer- und Systemereignissen unterschieden. Systemereignisse sind Ereignisse, die von den Analysen oder der Software selbst eingezeichnet werden. Benutzer-Ereignisse sind vom Benutzer eingezeichnete Ereignisse.

Folgende Regeln gelten bezüglich der Bewertung von Konfliktfällen: Benutzerereignisse sind immer höher priorisiert als Systemereignisse, wenn sie zeitlich parallel sind und sich nicht tolerieren. Beide Ereignisse bleiben gespeichert. Beim Zurückkehren auf den Zustand nach der auto Analyse müssen alle Benutzerereignisse gelöscht werden. Parallele Benutzerereignisse überschreiben sich gegenseitig. Parallele Systemereignisse überschreiben sich ebenfalls gegenseitig. Ebenso verhält es sich mit Bemerkungen.

Um sicherstellen zu können, dass Ereignisse, die der Benutzer schon manuell angelegt oder editiert hat, bei der automatischen Editierung nicht überschrieben werden, wird das Konzept der "User-Ereignisse" eingeführt. User-Ereignisse werden nicht bei automatischem Abgleich nach Editiervorgängen überschrieben oder geändert. Ereignisse, die für den Bericht als Beispiel ausgewählt werden, werden als User-Ereignis deklariert, damit sie geschützt sind.

Ereignisse, die im Bericht (als EKG-Strip) normgerecht ausgedruckt werden, werden vor Änderungen durch die automatische Editierung geschützt (z.B. als User-Ereignisse). Zeitlich gekoppelte Schlaf- und EKG-Ereignisse, werden automatisch angezeigt und in der EKG-Spur markiert.

Der von der erfindungsgemäßen Vorrichtung erstellte. Bericht umfasst folgende Informationen alternativ und/oder ergänzend:
- Patientenidentifikation in jeder Ansicht
- Alle vom Benutzer gewählten Parameter (Brady/Tachykardiegrenze, Pausenlänge, Kanäle)
- niedrigste, mittlere und höchste Herzfrequenz
- Gesamtzahl aller erkannten Herzschläge
- Ausgewertete Gesamtzeit
- Anzahl aller einzelnen SVE, gepaarten SVE und SVE-Folgen, SVE-Folgen über Gesamtzeitraum und je Stunde
- Dauer oder Anzahl Schläge je SVT
- Anzahl aller einzelnen VE, gepaarten VE und VT über Gesamtzeitraum und je Stunde
- Dauer oder Anzahl Schläge je VT
- Stundenübersicht über Bradykardieepisoden (< Grenzfrequenz und > 15 s)
- Liste der Bradykardieepisoden mit Länge und HF
- Anzahl Lage und Dauer aller Pausen
- Versatz zwischen den Kanälen kleiner 0.5 mm
- Datum, Uhrzeit und Dauer der Messung
- Patientendaten, Indikation, Medikation, Diagnose,
- Graph Herzfrequenz über die Zeit
- Min/max HR in Stundentabelle
- HRV: Indizes Zeitbereich
- HRV: SDNN-i als Graph
- HRV: NN als Graph
- HRV: Poincaré-Plot
- HRV: Indizes Frequenzbereich:
- HRV: Leistungsspektrum als Graph
- Relativer Anteil Dauer AA/Brady/Tachykardie gesamt und nach Stunden
- Verzeichnis der im Bericht enthaltenen EKG-Sequenzen
- Tabelle/Darstellung assoziierter respiratorischer und EKG-Ereignisse

Die erfindungsgemäße Vorrichtung kann in folgenden Modi betrieben werden:
1. Volles 24h-Langzeit-EKG mit Minimalanalyse + HRV (heart rate variability)
   ∘ Übersicht mit Verbindung Schlaf EKG
   ∘ Vollständiger, normgerechter EKG-Bericht mit HRV-Ergebnissen
   ∘ Ereigniseditierung mit Ausschnitt
   ∘ Schlageditierung mit Ausschnitt
   ∘ Ereignismarkierung und -editierung im Signal in der Übersicht
   ∘ EKG-Vollansicht
2. Nachtanalyse (ohne 24h-EKG-Aufzeichnung)
   ∘ Übersicht mit Verbindung Schlaf EKG
   ∘ (Vollständiger, normgerechter) EKG-Bericht mit HRV-Ergebnissen
   ∘ Ereigniseditierung mit Ausschnitt
   ∘ Ereignismarkierung und -editierung im Signal in der Übersicht
   ∘ Schlageditierung mit Signal-Ausschnitt
3. Nachtanalyse, kleine Version
   ∘ Übersicht mit Verbindung Schlaf EKG
   ∘ Ereignismarkierung und -editierung im Signal in der übersicht
   ∘ EKG-Berichtsteil, nicht normgerecht, (mit HRV)
   ∘ Ereigniseditierung mit Ausschnitt
   ∘ Ereignismarkierung und -editierung im EKG-Signal

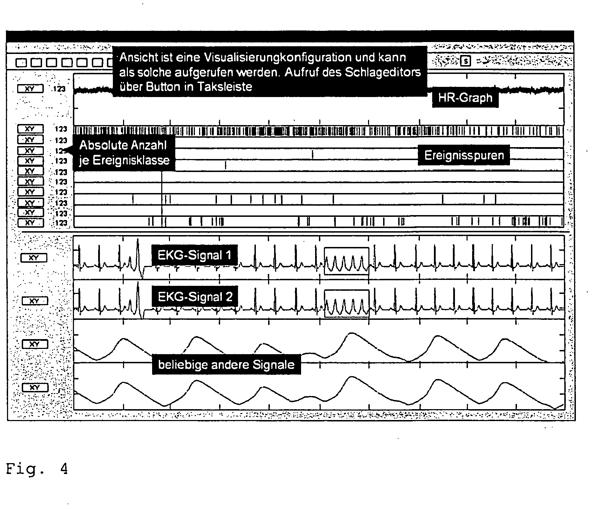

Figur 4 zeigt die Oberfläche der Software zur Auswertung / Verwaltung der registrierten Daten. Erfindungsgemäß ist erstmals die Auswertung und Zuordnung von EKG-Signalen und atmungsabhängigen Signalen, beispielsweise in Form von P(S)G-Daten, möglich. Die Software läuft entweder als Firmware im Gerät oder auf einem PC, an den die Daten zur Auswertung übermittelt werden. Die Software bietet eine Übersicht über die gesamte Aufzeichnung und ermöglicht den Vergleich der EKG-Daten mit den P(S)G-Daten. Der Vergleich ist für den Benutzer übersichtlich in einer Ansicht, beispielsweise auf einer DIN A4 - Seite, dargestellt. Die verschiedenen Kanäle sind untereinander angeordnet und zeitlich zueinander korreliert, so dass der Vergleich der EKG-Signale und der atmungsabhängigen Signalen einen Rückschluß auf Ereignisse zulässt, die ebenfalls in dem Hauptfenster dargestellt werden.

Dadurch ist es erstmals möglich, Ereignisse wie beispielsweise eine Arrythmie des Herzschlages, mit Atmungsdaten zu vergleichen, um darüber Rückschlüsse auf die Ursache der Arrythmie zu ziehen. Tritt eine Arrythmie beispielsweise immer dann auf, wenn auch Atmungsereignisse registriert werden, ist die Arrythmie vermutlich nicht herzbedingt, vielmehr liegt eine Atmungsstörung vor, die sich auf das Herz auswirkt. Es ist eine unmittelbare Auswertung vorgesehen, damit das Gerät unmittelbar auf Ereignisse reagieren kann und beispielsweise atmungsbedingte Arrythmien oder Ischämien durch Anpassung des Therapiedrucks eines Beatmungsgerätes und/oder durch Sauerstoffgabe therapieren kann. Das Gerät gibt ebenfalls einen Alarm aus, wenn definierbar schwere Ereignisse auftreten.

Neben einem Hauptfenster gibt es ein weiteres, konfigurierbares Übersichtsfenster.

Im Übersichtsfenster kann die Herzrate über die gesamte Aufzeichnungsdauer angezeigt werden. Alternativ können Herzrate und die P(S)G-Aufzeichnung angezeigt werden. Automatisch erkannte Ereignisse oder vom Benutzer definierte Ereignisse werden als Ereignisspuren, in Form einer strichartigen Markierung für jedes Ereignis, über die gesamte Aufzeichnung oder Nacht dargestellt. Die absolute Anzahl aller Ereignisse über die Auswertezeit ist anzeigbar. Ein Hypnogramm kann parallel angezeigt werden. Leere Ereignisspuren werden ausgeblendet.

Im Hauptfenster werden die EKG-Signale beider Kanäle für ein wählbares Zeitintervall dargestellt. Zusätzlich sind beliebige andere Signale, wie beispielsweise Atemfluss oder SpO2, unter den EKG-Signalen darstellbar.

Es erfolgt eine automatische oder vom Benutzer vorzunehmende Ereignismarkierung im EKG. Assoziierte Ereignisse wie beispielsweise Entsättigungen und Pausen, werden ebenfalls dargestellt.

Das Übersichtsfenster ist konfigurierbar, Signalspuren, Hypnogramm und HF-Trend sowie beliebige Signale sind darstellbar.

Im Berichtsfenster der Software ist eine editierbare Maske für Angaben zu Medikation, Schrittmacher, Indikation und Diagnose, mit tabellarischer Übersicht der Ergebnisse des automatischen Befunds darstellbar.

Erfindungsgemäß ist eine Konfiguration zur Aufzeichnung der Signale vorgesehen. Der Benutzer legt vor Messbeginn fest, welche Kanäle wann im Zeitraum der Messung und welche in welchem Zeitabschnitt in der Nacht aufgezeichnet werden. Es besteht eine freie Konfigurierbarkeit eines Intervalles für Langzeit-EKG-Messung und eine freie Konfigurierbarkeit des PG/PSG-Abschnittes.

Eine wesentliche Erfindungskomponente ist darin zu sehen, daß ein Gerät bereitstellt wird, das vollautomatisch entweder selbst Diagnoseergebnisse liefert oder einem Arzt zur Unterstützung der Stellung einer Diagnose strukturiert die erforderlichen Daten zur Verfügung stellt. Die Abspeicherung der Daten der einzelnen Meßkanäle unter Berücksichtigung zugeordneter Zeitinformationen ermöglicht es, bei einer späteren Auswertung alle Meßinformationen unter Berücksichtigung der zugeordneten Zeitinformationen auszuwerten.

Es wird erfindungsgemäß ein Gerät bereitgestellt, das sowohl die erforderlichen Messungen durchführt, die Auswertungen vornimmt und die Auswertungsergebnisse ausgibt bzw. anzeigt. Gemäß einer zusätzlichen Ausführungsform kann das Gerät auch direkt mit dem Patienten in Verbindung stehende Geräte steuern. Beispielsweise kann dies die Steuerung eines Beatmungsgerätes oder eines Herzschrittmachers sein.

Die unmittelbar auf den Patienten einwirkenden Funktionen des Gerätes können von einem behandelnden Arzt deaktivierbar sein, so daß eine volle Funktionskontrolle durch den Arzt gegeben ist.

## Patentansprüche

1. Datenerfassungsanordnung mit Sensoren und Auswertungseinrichtung, zur Überwachung zumindest zweier physiologischer Parameter (1) eines Patienten, nämlich mindestens eines Herzaktionsparameters mittels eines Messverfahrens ausgewählt aus der Gruppe
a) EKG (Elektrokardiogramm) mit mindestens 2 Kanälen und einer Schrittmachererkennung,
b) Fingerpulsamplitude (FPA),
und mindestens eines Atmungsparameters mittels eines Messverfahrens ausgewählt aus einer Gruppe
c) Pulsoxymetrie,
d) respiratorische Polygraphie,
e) Polysomnografie
wobei die Datenerfassungsanordnung jeden physiologischen Parameter (1) in einem separaten Kanal aufzeichnet und aufeinanderfolgende Werte der physiologischen Variablen speichert zum Gewinnen einer graphischen Darstellung oder Auswertung der gespeicherten Werte, wobei eine Takteinrichtung ein Zeitsignal erzeugt und eine Steuereinrichtung der Datenerfassungsanordnung während des Zeitsignals alle Kanäle der Datenerfassungsanordnung aufzeichnet und jedem Datensignal ein Zeitsignal zuordnet und bei der zur Übertragung der Meßdaten der verschiedenen Sensoren an die Auswertungseinrichtung (17) getrennte Kanäle verwendet sind, wobei zur Konfigurierung eines Zeitraumes der Erfassung und Aufzeichnung innerhalb der gesamten Meßdauer für jeden Kanal einzeln oder für mehrere Kanäle gebündelt eine Konfigurationsmöglichkeit verwendet ist, wobei die Vorrichtung zumindest eine Sensoreinrichtung (4-8) aufweist, die zumindest einen Herzaktionsparameter, einen Atmungsparameter und einen Schlafparameter ermittelt, sowie dass eine Zeitbestimmungseinrichtung, die eine Bestimmung von Zeitinformationen ermöglicht und eine Speichereinrichtung, die die ermittelten Biosignale und Zeitinformationen wieder abrufbar speichert, verwendet sind, **dadurch gekennzeichnet, dass** die Vorrichtung eine Vergleichseinrichtung aufweist, die auch nächtliche Atmungsstörungen sowie EKG-Anomalien erkennt, sowie die EKG-Anomalien (Arrhythmien etc.) Wach- und Schlaf-Zustand zuordnen kann sowie zeitliche Assoziationen zwischen EKG-Anomalien und Atmungsstörungen herstellen kann, um einen ursächlichen Zusammenhang von beiden untersuchen zu können, dass die Messdaten der verschiedenen Sensoren über getrennte Kanäle einer gemeinsamen Auswertungseinrichtung bereitstellbar sind, dass die Messdaten von der Vorrichtung zur Erfassung der physiologischen Parameter (1) durch eine Auswertungseinrichtung (17) analysierbar sind und im Bereich der Speichereinrichtung (16) gespeichert werden und über Schnittstellen die Daten an externe Geräte wie einen PC (18) oder ein Beatmungsgerät-Gerät (19) weitergegeben werden können, dass die Auswertung am PC und/oder im Gerät erfolgt und dass die Oberfläche der Software zur Auswertung / Verwaltung der registrierten Daten derart ausgebildet ist, dass die Auswertung und Zuordnung von EKG-Signalen und atmungsabhängigen Signalen, beispielsweise in Form von P(S)G-Daten, möglich ist und dass die Software entweder als Firmware im Gerät oder auf einem PC ablauffähig ist, an den die Daten zur Auswertung übermittelt werden und dass die Software eine Übersicht über die gesamte Aufzeichnung bietet und den Vergleich der EKG-Daten mit den P(S)G-Daten ermöglicht, wobei der Vergleich für den Benutzer übersichtlich in einer Ansicht, beispielsweise auf einer DIN A4 - Seite, darstellbar ist und dass die verschiedenen Kanäle untereinander angeordnet und zeitlich zueinander korreliert sind, so dass der Vergleich der EKG-Signale und der atmungsabhängigen Signalen einen Rückschluss auf Ereignisse zulässt, die ebenfalls in dem Hauptfenster dargestellbar sind.

2. Datenerfassungsanordnung nach Anspruch 1, **dadurch gekennzeichnet, dass** eine Verbindungseinrichtung zum wahlweisen Anschließen der Datenerfassungsanordnung an einen PC oder an ein Therapiegerät verwendet ist.

3. Datenerfassungsanordnung nach Anspruch 1 oder 2, **dadurch gekennzeichnet, dass** eine Energieversorgung zur netzunabhängigen Zufuhr elektrischer Energie zu der Anordnung vorgesehen ist und dass mit der Energieversorgung eine Energieprüfeinrichtung verbunden ist, die ein Niedrigenergiesignal erzeugt, wenn eine elektrische Kenngröße der elektrischen Versorgungsenergie kleiner als ein vorbestimmter Pegel ist.

4. Datenerfassungsanordnung nach einem der vorhergehenden Ansprüche, **dadurch gekennzeichnet, dass** ein Analysator eine unmittelbare Auswertung der Daten durchführt und definierbare Ereignisse ermittelt.

5. Datenerfassungsanordnung nach einem der vorhergehenden Ansprüche, **dadurch gekennzeichnet, dass** ein Alarm ausgegeben wird, wenn definierbar schwere Ereignisse auftreten.

6. Datenerfassungsanordnung nach einem der vorhergehenden Ansprüche, **dadurch gekennzeichnet, dass** bei Auftreten definierter Ereignisse ein Steuersignal für angeschlossene Therapiegeräte generiert wird.

7. Datenerfassungsanordnung nach Anspruch 6, **dadurch gekennzeichnet, dass** das Steuersignal ein Beatmungsgerät beeinflusst, den Therapiedruck anzupassen.

8. Datenerfassungsanordnung nach einem der vorhergehenden Ansprüche, **dadurch gekennzeichnet, dass** das Steuersignal ein Sauerstoffdosiergerät beeinflusst, eine erhöhte Sauerstoffkonzentration und/oder eine erhöhte Sauerstoffmenge bereitzustellen.

9. Datenerfassungsanordnung nach einem der Ansprüche 1 bis 8, **dadurch gekennzeichnet, dass** eine digitale Datenspeicherung realisiert ist.

10. Datenerfassungsanordnung nach einem der Ansprüche 1 bis 8, **dadurch gekennzeichnet, dass** eine analoge Datenspeicherung realisiert ist.

11. Datenerfassungsanordnung nach einem der Ansprüche 1 bis 10, **dadurch gekennzeichnet, dass** die Daten zeitlich zueinander korreliert sind.

12. Datenerfassungsanordnung nach einem der Ansprüche 1 bis 11, **dadurch gekennzeichnet, dass** ein Analysator zum Vergleich von Ereignissen, wie beispielsweise eine Arrythmie des Herzschlages mit Atmungsdaten, verwendet ist, um darüber Rückschlüsse auf die Ursache der Arrythmie zu ziehen.

13. Datenerfassungsanordnung nach einem der Ansprüche 1 bis 12, **dadurch gekennzeichnet, dass** nach Ablauf der beiden Einzel-Auswertungen das System eine Aussage über die zeitliche und/oder ursächliche Verknüpfung von EKG- sowie somno-respiratorischen Ereignissen bereitstellt.

14. Datenerfassungsanordnung nach einem der Ansprüche 1 bis 13, **dadurch gekennzeichnet, dass** eine Analyse der Meßdaten durch statistische Aussagen über die Häufigkeit von Assoziationen zwischen EKG- und somno-respiratorischen Ereignissen im Bericht, zumindest beschränkt auf solche Verknüpfungen die plausibel erscheinen, durchführbar ist.

15. Datenerfassungsanordnung nach einem der Ansprüche 1 bis 14, **dadurch gekennzeichnet, dass** eine Analyse der Meßdaten durch Differenzierung der Aussagen über EKG-Ereignisse und Herzfrequenzvariabilität nach Schlafstadien im Bericht, bereitgestellt ist.

16. Datenerfassungsanordnung nach einem der Ansprüche 1 bis 15, **dadurch gekennzeichnet, dass** nur anhand des EKGs REM- und Nicht-REM-Phasen voneinander unterschieden werden.

17. Verfahren zur Darstellung und Auswertung von EKG-Signalen und atmungsabhängigen Signalen in Form einer vergleichenden Darstellung der verschiedenen Kanäle miteinander unter Verwendung einer Vorrichtung nach Anspruch 1, wobei die Daten zeitlich zueinander korreliert abgespeichert werden, sowie bei dem für die Erkennung eines Artefaktes eines Herzschrittmachers eine EKG-Ableitung durchgeführt wird, wobei eine kontinuierliche Aufzeichnung über zumindest sechs Stunden bei simultaner und mindestens zwei-kanaliger EKG-Ableitung durchgeführt wird, und bei dem zur Übertragung der Meßdaten der verschiedenen Sensoren an die Auswertungseinrichtung (17) getrennte Kanäle verwendet werden, wobei zur Konfigurierung eines Zeitraumes der Erfassung und Aufzeichnung innerhalb der gesamten Meßdauer für jeden Kanal einzeln oder für mehrere Kanäle gebündelt eine Konfigurationsmöglichkeit verwendet wird, wobei für den EKG-Kanal eine Schrittmachererkennung derart vorgesehen ist, dass wenn der Patient einen Herzschrittmacher hat, das Artefakt des Schrittmachers durch das EKG erkannt wird, wobei dazu zwei Möglichkeiten vorgesehen sind, nämlich
a) Erhöhen der Abtastrate über 2 KHz bei einer digitalen Signalaufzeichnung oder
b) Schaltung (analog), die am Ausgang einen zeitlich langen Spannungswert bereitstellt, der auch bei einer Abtastrate von 256 Hz oder niedriger als 2 KHz ermittelt werden kann.

18. Verfahren nach Anspruch 17, **dadurch gekennzeichnet, dass** nach Ablauf der beiden Einzel-Auswertungen das System eine Aussage über die zeitliche Verknüpfung von EKG- sowie somno-respiratorischen Ereignissen bereitstellt.

19. Verfahren nach Anspruch 18, **dadurch gekennzeichnet, dass** die Aussage durch Übersichtsdarstellungen mit zeitlicher Markierung sämtlicher Ereignisse am Bildschirm und im Bericht bereitgestellt wird.

20. Verfahren nach Anspruch 18, **dadurch gekennzeichnet, dass** die Aussage durch statistische Auswertungen über die Häufigkeit von Assoziationen zwischen EKG- und somno-respiratorischen Ereignissen im Bericht, zumindest beschränkt auf solche Verknüpfungen die plausibel erscheinen, bereitgestellt wird.

21. Verfahren nach Anspruch 18, **dadurch gekennzeichnet, dass** die Aussage durch Differenzierung der Aussagen über EKG-Ereignisse und Herzfrequenzvariabilität nach Schlafstadien im Bericht bereitgestellt wird.

22. Verfahren nach Anspruch 18, **dadurch gekennzeichnet, dass** in der Aussage nur anhand des EKGs REM- und Nicht-REM-Phasen voneinander unterschieden werden.

## Claims

1. Data capture arrangement comprising sensors and an evaluation device, for monitoring at least two physiological parameters (1) of a patient, specifically at least one heartbeat parameter by means of a measurement method selected from the group
a) ECG (electrocardiogram) with at least 2 channels and a pacemaker identification,
b) finger pulse amplitude (FPA),
and at least one respiration parameter by means of a measurement method selected from a group
c) pulse oximetry,
d) respiratory polygraphy,
e) polysomnography,
wherein the data capture arrangement records each physiological parameter (1) in a separate channel and stores successive values of the physiological variable for the purposes of obtaining a graphical representation or evaluation of the stored values, wherein a clock device produces a time signal and a control device of the data capture arrangement records all channels of the data capture arrangement during the time signal and assigns each data signal a time signal and in which separate channels are used for transmitting the measured data of the various sensors to the evaluation device (17), wherein, for the purposes of configuring a time period of capture and recording within the overall measurement duration, a configuration option is used for each channel individually or for a plurality of channels bundled together, wherein the apparatus has at least one sensor device (4-8), which ascertains at least one heartbeat parameter, one respiration parameter and one sleep parameter and in that a time determination device, which facilitates the determination of time information items, and a storage device, which stores the ascertained bio signals and time information items in recallable fashion, are used, **characterized in that** the apparatus has a comparison device which also identifies nocturnal respiration disorders and ECG anomalies and which is able to assign the ECG anomalies (arrhythmia, etc.) an awake state and a sleep state and which can establish temporal associations between ECG anomalies and respiration disorders in order to be able to examine a causal relationship of both, **in that** the measured data of the various sensors are providable to a common evaluation device via separate channels, **in that** the measured data from the apparatus for capturing the physiological parameters (1) are analysable by an evaluation device (17) and stored in the region of the storage device (16) and the data can be transmitted to external appliances such as a PC (18) or a ventilator appliance (19) by interfaces, **in that** the evaluation is implemented on the PC and/or in the appliance and **in that** the interface of the software for evaluating/managing the registered data is embodied in such a way that the evaluation and assignment of ECG signals and respiration-dependent signals, for example in the form of P(S)G data, is possible and **in that** the software is executable either as firmware in the appliance or on the PC, to which the data are transmitted for evaluation purposes, and **in that** the software offers an overview of the entire recording and facilitates the comparison of the ECG data with the P(S)G data, wherein the comparison is representable in clear fashion to the user in a view, for example on a DIN A4 sheet, and **in that** the various channels are arranged below one another and correlated in time with respect to one another such that the comparison of the ECG signals and the respiration-dependent signals allow deductions to be made about events, which are likewise representable in the main window.

2. Data capture arrangement according to Claim 1, **characterized in that** use is made of a connecting device for selectively connecting the data capture arrangement to a PC or to a therapy appliance.

3. Data capture arrangement according to Claim 1 or 2, **characterized in that** a power supply is provided for mains-independent supply of electric power to the arrangement and **in that** a power monitoring device is connected to the power supply, said power monitoring device producing a low-power signal if an electric characteristic of the electric power supply is lower than a predetermined level.

4. Data capture arrangement according to any one of preceding claims, **characterized in that** an analyser carries out an immediate evaluation of the data and ascertains definable events.

5. Data capture arrangement according to any one of the preceding claims, **characterized in that** an alarm is output if definably serious events occur.

6. Data capture arrangement according to any one of the preceding claims, **characterized in that** a control signal for connected therapy appliances is generated following the occurrence of defined events.

7. Data capture arrangement according to Claim 6, **characterized in that** the control signal influences a ventilator apparatus so to adjust the therapy pressure.

8. Data capture arrangement according to any one of the preceding claims, **characterized in that** the control signal influences an oxygen metering appliance so as to provide an elevated oxygen concentration and/or an increased amount of oxygen.

9. Data capture arrangement according to any one of Claims 1 to 8, **characterized in that** digital data storage is realized.

10. Data capture arrangement according to any one of Claims 1 to 8, **characterised in that** analogue data storage is realized.

11. Data capture arrangement according to any one of Claims 1 to 10, **characterized in that** the data are correlated with one another in time.

12. Data capture arrangement according to any one of Claims 1 to 11, **characterized in that** an analyser is used for comparing events, such as an arrhythmia of the heartbeat with respiration data, for example, in order thereby to draw conclusions about the cause of the arrhythmia.

13. Data capture arrangement according to any one of Claims 1 to 12, **characterized in that**, once the two individual evaluations have been carried out, the system provides a statement about the temporal and/or causal link of ECG and sleep-respiratory events.

14. Data capture arrangement according to any one of Claims 1 to 13, **characterized in that** an analysis of the measured data is performable by way of statistical statements about the frequency of associations between ECG and sleep-respiratory events in the report, at least restricted to those links that appear plausible.

15. Data capture arrangement according to any of Claims 1 to 14, **characterized in that** an analysis of the measured data is provided by differentiating the statements about ECG events and heart rate variability according to sleep stages in the report.

16. Data capture arrangement according to any one of Claims 1 to 15, **characterized in that** REM and non-REM phases are distinguished from one another on the basis of the ECG only.

17. Method for representing and evaluating ECG signals and respiratory-dependent signals in the form of a comparative representation of the various channels with one another using an apparatus according to Claim 1, wherein the data are stored with temporal correlation to one another and in which an ECG recording is performed for identifying an artefact of the cardiac pacemaker, wherein a continuous recording is carried out over at least six hours with a simultaneous and at least two-channel ECG recording, and in which separate channels are used to transmit the measured data of the various sensors to the evaluation device (17), wherein a configuration option is used for configuring a time period for capturing and recording within the entire measurement duration, either for each channel individually or for a plurality of channels bundled together, wherein, for the ECG channel, a pacemaker identification is provided in such a way that, if the patient has a cardiac pacemaker, the artefact of the pacemaker is identified by the ECG, wherein two options are provided to this end, specifically
a) increasing the sampling rate above 2 kHz in the case of a digital signal recording or
b) an (analogue) circuit, which provides a temporally long voltage value at an output, which can be ascertained even in the case of a sampling rate of 256 Hz or less than 2 kHz.

18. Method according to Claim 17, **characterized in that**, once the two individual evaluations have been carried out, the system provides a statement about the temporal link of ECG and sleep-respiratory events.

19. Method according to Claim 18, **characterized in that** the statement is provided by overview representations with temporal marking of all events on the screen and in the report.

20. Method according to Claim 18, **characterized in that** the statement is provided by statistical evaluations about the frequency of associations between ECG and sleep-respiratory events in the report, at least restricted to those links that appear plausible.

21. Method according to Claim 18, **characterized in that** the statement is provided by differentiating the statements about ECG events and heart rate variability according to sleep stages in the report.

22. Method according to Claim 18, **characterized in that** REM and non-REM phases are distinguished from one another in the statement on the basis of the ECG only.

## Revendications

1. Dispositif d'acquisition de données comprenant des capteurs et un moyen d'évaluation, ledit dispositif d'acquisition de données étant destiné à surveiller au moins deux paramètres physiologiques (1) d'un patient, à savoir au moins un paramètre d'activité cardiaque au moyen d'un procédé de mesure sélectionné dans le groupe comprenant
a) un ECG (électrocardiogramme) comportant au moins 2 canaux et un moyen de détection de stimulateur cardiaque,
b) une amplitude de pouls digital (FPA),
et au moins un paramètre respiratoire au moyen d'un procédé de mesure sélectionnée dans un groupe comprenant
c) une oxymétrie de pouls,
d) une polygraphie respiratoire,
e) une polysomnographie
le dispositif d'acquisition de données enregistrant chaque paramètre physiologique (1) dans un canal séparé et mémorisant des valeurs successives des grandeurs physiologiques pour obtenir une représentation graphique ou une évaluation des valeurs mémorisées, un moyen d'horloge générant un signal de temps et un moyen de commande du dispositif d'acquisition de données enregistrant tous les canaux du dispositif d'acquisition de données pendant le signal de temps et associant un signal de temps à chaque signal de données et des canaux séparés étant utilisés pour transmettre les données de mesure des divers capteurs au moyen d'évaluation (17), une option de configuration étant utilisée pour configurer un intervalle de temps d'acquisition et d'enregistrement pendant toute la durée de mesure pour chaque canal individuellement ou pour plusieurs canaux en groupe, le dispositif comprenant au moins un moyen capteur (4-8), qui détermine au moins un paramètre d'activité cardiaque, un paramètre de respiration et un paramètre de sommeil, ainsi qu'un moyen de détermination de temps qui permet une détermination d'informations de temps et un moyen de mémorisation qui mémorise de manière récupérable les signaux biologiques déterminés et les informations de temps, **caractérisé en ce que** le dispositif comporte un moyen de comparaison qui détecte également des troubles respiratoires nocturnes et des anomalies de l'ECG, et qui peut associer les anomalies de l'ECG (arythmies, etc.) à un état de veille et de sommeil et qui peut établir des associations temporelles entre les anomalies de l'ECG et les troubles respiratoires pour pouvoir étudier un lien de causalité entre les deux, **en ce que** les données de mesure des différents capteurs peuvent être fournies par des canaux séparés d'un moyen d'évaluation commun, **en ce que** les données de mesure provenant du dispositif d'acquisition de paramètres physiologiques (1) peuvent être analysées par un moyen d'évaluation (17) et sont mémorisées dans la région du moyen de mémorisation (16) et les données peuvent être transmises par le biais d'interfaces à des appareils externes tels qu'un PC (18) ou un appareil respiratoire (19), **en ce que** l'évaluation est effectuée sur le PC et/ou dans l'appareil et **en ce que** la surface du logiciel d'évaluation/de gestion des données enregistrées est conçue de telle sorte que l'évaluation et l'association des signaux d'ECG et des signaux dépendant de la respiration soient possibles, par exemple sous la forme de données P(S)G, et **en ce que** le logiciel est exécutable sous forme de micrologiciel dans l'appareil ou sur un PC, auquel les données sont transmises pour évaluation et **en ce que** le logiciel offre une vue d'ensemble de tout l'enregistrement et permet la comparaison des données d'ECG avec les données P(S)G, la comparaison pouvant être représentée clairement pour l'utilisateur dans une vue, par exemple sur une page DIN A4, et **en ce que** les différents canaux sont disposés les uns au-dessous des autres et sont corrélés temporellement les uns aux autres de sorte que la comparaison des signaux d'ECG et des signaux dépendant de la respiration permet de tirer des conclusions sur les événements qui peuvent également être représentés dans la fenêtre principale.

2. Dispositif d'acquisition de données selon la revendication 1, **caractérisé en ce qu'**un moyen de liaison est utilisé pour relier sélectivement le dispositif d'acquisition de données à un PC ou à un appareil thérapeutique.

3. Dispositif d'acquisition de données selon la revendication 1 ou 2, **caractérisé en ce qu'**il est prévu une alimentation en énergie destinée à amener, indépendamment du réseau, de l'énergie au dispositif, et **en ce qu'**un dispositif de contrôle d'énergie est relié à l'alimentation en énergie, lequel génère un signal de faible énergie lorsqu'un paramètre électrique de l'énergie d'alimentation électrique est inférieur à un niveau prédéterminé.

4. Dispositif d'acquisition de données selon l'une des revendications précédentes, **caractérisé en ce qu'**un analyseur effectue une évaluation immédiate des données et détermine des événements définissables.

5. Dispositif d'acquisition de données selon l'une des revendications précédentes, **caractérisé en ce qu'**une alarme est émise lorsque des événements importants définissables se produisent.

6. Dispositif d'acquisition de données selon l'une des revendications précédentes, **caractérisé en ce que**, lorsque des événements définis se produisent, un signal de commande est généré à destination d'appareils thérapeutiques.

7. Dispositif d'acquisition de données selon la revendication 6, **caractérisé en ce que** le signal de commande influe sur un appareil respiratoire pour ajuster la pression thérapeutique.

8. Dispositif d'acquisition de données selon l'une des revendications précédentes, **caractérisé en ce que** le signal de commande influe sur un appareil de dosage d'oxygène pour fournir une concentration en oxygène accrue et/ou une quantité d'oxygène accrue.

9. Dispositif d'acquisition de données selon l'une des revendications 1 à 8, **caractérisé en ce qu'**une mémorisation de données numérique est réalisée.

10. Dispositif d'acquisition de données selon l'une des revendications 1 à 8, **caractérisé en ce qu'**une mémorisation de données analogique est réalisée.

11. Dispositif d'acquisition de données selon l'une des revendications 1 à 10, **caractérisé en ce que** les données sont corrélées temporellement les unes aux autres.

12. Dispositif d'acquisition de données selon l'une des revendications 1 à 11, **caractérisé en ce qu'**un analyseur est utilisé pour comparer des événements, tels qu'une arythmie du rythme cardiaque, avec des données de respiration afin de tirer des conclusions sur la cause de l'arythmie.

13. Dispositif d'acquisition de données selon l'une des revendications 1 à 12, **caractérisé en ce que**, lorsque deux évaluations individuelles sont terminées, le système donne des informations sur le lien temporel et/ou causal entre des événements d'ECG et des événements somno-respiratoires.

14. Dispositif d'acquisition de données selon l'une des revendications 1 à 13, **caractérisé en ce qu'**une analyse des données de mesure peut être effectuée par des informations statistiques sur la fréquence des associations entre des événements d'ECG et des événements somno-respiratoires dans le rapport, au moins de façon limitée à de tels liens qui semblent plausibles.

15. Dispositif d'acquisition de données selon l'une des revendications 1 à 14, **caractérisé en ce qu'**une analyse des données de mesure par différenciation des informations sur les événements d'ECG et la variabilité de la fréquence cardiaque après les étapes de sommeil peut être fournie dans le rapport.

16. Dispositif d'acquisition de données selon l'une des revendications 1 à 15, **caractérisé en ce que** les phases REM et non-REM sont distinguées l'une de l'autre uniquement sur la base de l'ECG.

17. Procédé de représentation et d'évaluation de signaux ECG et de signaux dépendant de la respiration sous la forme d'une représentation comparative des différents canaux entre eux à l'aide d'un dispositif selon la revendication 1, les données étant mémorisées de manière corrélée temporellement les unes aux autres, et une dérivation d'ECG étant effectuée pour la détection d'un artefact d'un stimulateur cardiaque, un enregistrement continu étant effectué pendant au moins six heures avec une dérivation d'ECG simultanée et au moins à deux canaux, et des canaux séparés étant utilisés pour transmettre les données de mesure des divers capteurs au moyen d'évaluation (17), une option de configuration étant utilisée pour configurer un intervalle de temps d'acquisition et d'enregistrement pendant toute la durée de mesure pour chaque canal individuellement ou pour plusieurs canaux en groupe, une détection de stimulateur cardiaque étant prévue pour le canal ECG de manière à ce que, lorsque le patient est équipé d'un stimulateur cardiaque, l'artefact de stimulateur cardiaque est reconnu par l'ECG, deux options étant pour cela prévues, à savoir
a) augmentation de la vitesse d'exploration au-dessus de 2 KHz dans le cas d'un enregistrement de signal numérique ou
b) circuit (analogique) qui fournit en sortie une valeur de tension temporellement longue qui peut être déterminée même à une vitesse d'exploration de 256 Hz ou inférieure à 2 KHz.

18. Procédé selon la revendication 17, **caractérisé en ce que**, lorsque les deux évaluations individuelles sont terminées, le système fournit une information sur le lien temporel entre des événements ECG et des événements somno-respiratoires.

19. Procédé selon la revendication 18, **caractérisé en ce que** l'information est fournie par des représentations d'ensemble avec marquage temporel de tous les événements sur l'écran et dans le rapport.

20. Procédé selon la revendication 18, **caractérisé en ce que** les évaluations statistiques sur la fréquence des associations entre les événements d'ECG et les événements somno-respiratoires sont fournies dans le rapport, au moins de façon limitée à de tels liens paraissant plausibles.

21. Procédé selon la revendication 18, **caractérisé en ce que** l'information est fournie dans le rapport par différenciation des informations sur les événements d'ECG et la variabilité de la fréquence cardiaque après les stades de sommeil.

22. Procédé selon la revendication 18, **caractérisé en ce que** les phases REM et non-REM sont distinguées l'une de l'autre dans l'information uniquement sur la base de l'ECG.
